# EUROPEAN PATENT APPLICATION

(11) **EP 1 889 906 A1**
(43) Date of publication of application: **20.02.2008**
(21) Application number: 06747085.6
(22) Date of filing: 01.06.2006
(51) Int. Cl.: C12N 15/00, C12N 1/15, C12N 1/19, C12N 1/21, C12N 5/10, C12N 15/09, C12P 21/02

(54) **HIGH-COPY-NUMBER, HIGH-EXPRESSION VECTOR HAVING METHIONINE AMINOPEPTIDASE GENE THEREIN**

(30) Priority: 03.06.2005 JP 2005164552
(71) Applicant: NIPRO CORPORATION, Kita-ku, Osaka-shi, Osaka-fu, 531-8510 (JP)
(72) Inventor: HOASHI, Yohei, c/o NIPRO CORPORATION,, Osaka-shi, Osaka-fu 531-8510 (JP); UNO, Tadayuki, Osaka-fu 531-8510 (JP); KAI, Toshiya, c/o NIPRO CORPORATION,, Osaka-shi,Osaka-fu 531-8510 (JP); KATAYAMA, Naohisa, c/o NIPRO CORPORATION,, Osaka-shi,Osaka-fu 531-8510 (JP)
(74) Representative: Hiebl, Inge Elisabeth
(86) International application number: PCT/JP2006/310983
(87) International publication number: WO 2006/129751

(57) **Abstract**

Provided is a high-copy-number, high-expression vector capable of producing a protein having satisfactory functions and activity of the same level as that of a natural form of the protein, in a large quantity and in a simple manner. Also provided is a vector including: (A) a target gene or a cloning site of the target gene, (B) a sequence element necessary for the high copying of the target gene, (C) a sequence element necessary for the expression of the target gene and a methionine aminopeptidase gene; a method of producing the vector; a transformant having the vector introduced therein; and a process for producing a protein using the transformant.

SEQUENCE LISTING

<110> NIPRO CORPORATION

<120> High-copy-number, high-expression vector having methionine
aminopeptidase gene therein

<130> 18262EP

<140> EP 06 747 085.6
<141> 2006-06-01

<150> JP2005-164552
<151> 2005-06-03

<160> 4

<170> PatentIn version 3.1

<210> 1
<211> 3143
<212> DNA
<213> Homo sapiens

<400> 1
ctaaattgta agcgttaata ttttgttaaa attcgcgtta aatttttgtt aaatcagctc 60

attttttaac caataggccg aaatcggcaa aatcccttat aaatcaaaag aatagaccga 120

gatagggttg agtgttgttc cagtttggaa caagagtcca ctattaaaga acgtggactc 180

caacgtcaaa gggcgaaaaa ccgtctatca gggcgatggc ccactacgtg aaccatcacc 240

ctaatcaagt tttttggggt cgaggtgccg taaagcacta aatcggaacc ctaaagggag 300

cccccgattt agagcttgac ggggaaagcc ggcgaacgtg gcgagaaagg aagggaagaa 360

agcgaaagga gcgggcgcta gggcgctggc aagtgtagcg gtcacgctgc gcgtaaccac 420

cacacccgcc gcgcttaatg cgccgctaca gggcgcgtcc cattcgccat tcaggctgcg 480

caactgttgg gaagggcgat cggtgcgggc ctcttcgcta ttacgccagc tggcgaaagg 540

gggatgtgct gcaaggcgat taagttgggt aacgccaggg ttttcccagt cacgacgttg 600

taaaacgacg gccagtgagc gcgcgtaata cgactcacta tagggcgaat tggagctcca 660

ccgcggtggc ggccgctcta gaaataattt tgtttaactt taagaaggag atatacatat 720

ggctagcatg actggtggac agcaaatggg tcgcggatcc ggctgctaac aaagcccgaa 780

aggaagctga gttggctgct gccaccgctg agcaataact agcataaccc cttggggcct 840

ctaaacgggt cttgaggggt tttttgctga aaggaggaac tatatccgga attcgatatc 900

aagcttatcg ataccgtcga cctcgagggg gggcccggta cccagctttt gttcccttta 960

gtgagggtta attgcgcgct tggcgtaatc atggtcatag ctgtttcctg tgtgaaattg 1020

ttatccgctc acaattccac acaacatacg agccggaagc ataaagtgta aagcctgggg 1080

tgcctaatga gtgagctaac tcacattaat tgcgttgcgc tcactgcccg ctttccagtc 1140

gggaaacctg tcgtgccagc tgcattaatg aatcggccaa cgcgcgggga gaggcggttt 1200

gcgtattggg cgctcttccg cttcctcgct cactgactcg ctgcgctcgg tcgttcggct 1260
gcggcgagcg gtatcagctc actcaaaggc ggtaatacgg ttatccacag aatcagggga 1320
taacgcagga aagaacatgt gagcaaaagg ccagcaaaag gccaggaacc gtaaaaaggc 1380
cgcgttgctg gcgtttttcc ataggctccg cccccctgac gagcatcaca aaaatcgacg 1440
ctcaagtcag aggtggcgaa acccgacagg actataaaga taccaggcgt ttccccctgg 1500
aagctccctc gtgcgctctc ctgttccgac cctgccgctt accggatacc tgtccgcctt 1560
tctcccttcg ggaagcgtgg cgctttctca tagctcacgc tgtaggtatc tcagttcggt 1620
gtaggtcgtt cgctccaagc tgggctgtgt gcacgaaccc cccgttcagc ccgaccgctg 1680
cgccttatcc ggtaactatc gtcttgagtc caacccggta agacacgact tatcgccact 1740
ggcagcagcc actggtaaca ggattagcag agcgaggtat gtaggcggtg ctacagagtt 1800
cttgaagtgg tggcctaact acggctacac tagaaggaca gtatttggta tctgcgctct 1860
gctgaagcca gttaccttcg gaaaaagagt tggtagctct tgatccggca aacaaaccac 1920
cgctggtagc ggtggttttt ttgtttgcaa gcagcagatt acgcgcagaa aaaaaggatc 1980
tcaagaagat cctttgatct tttctacggg gtctgacgct cagtggaacg aaaactcacg 2040
ttaagggatt ttggtcatga gattatcaaa aaggatcttc acctagatcc ttttaaatta 2100
aaaatgaagt tttaaatcaa tctaaagtat atatgagtaa acttggtctg acagttacca 2160
atgcttaatc agtgaggcac ctatctcagc gatctgtcta tttcgttcat ccatagttgc 2220
ctgactcccc gtcgtgtaga taactacgat acgggagggc ttaccatctg gccccagtgc 2280
tgcaatgata ccgcgagacc cacgctcacc ggctccagat ttatcagcaa taaaccagcc 2340
agccggaagg gccgagcgca gaagtggtcc tgcaacttta tccgcctcca tccagtctat 2400
taattgttgc cgggaagcta gagtaagtag ttcgccagtt aatagtttgc gcaacgttgt 2460
tgccattgct acaggcatcg tggtgtcacg ctcgtcgttt ggtatggctt cattcagctc 2520
cggttcccaa cgatcaaggc gagttacatg atcccccatg ttgtgcaaaa aagcggttag 2580
ctccttcggt cctccgatcg ttgtcagaag taagttggcc gcagtgttat cactcatggt 2640
tatggcagca ctgcataatt ctcttactgt catgccatcc gtaagatgct tttctgtgac 2700
tggtgagtac tcaaccaagt cattctgaga atagtgtatg cggcgaccga gttgctcttg 2760
cccggcgtca atacgggata ataccgcgcc acatagcaga actttaaaag tgctcatcat 2820
tggaaaacgt tcttcggggc gaaaactctc aaggatctta ccgctgttga gatccagttc 2880
gatgtaaccc actcgtgcac ccaactgatc ttcagcatct tttactttca ccagcgtttc 2940
tgggtgagca aaaacaggaa ggcaaaatgc cgcaaaaaag ggaataaggg cgacacggaa 3000
atgttgaata ctcatactct tcctttttca atattattga agcatttatc agggttattg 3060
tctcatgagc ggatacatat ttgaatgtat ttagaaaaat aaacaaatag gggttccgcg 3120

cacatttccc cgaaaagtgc cac                                          3143

<210>  2
<211>  3967
<212>  DNA
<213>  Homo sapiens

<400>  2
ctaaattgta agcgttaata ttttgttaaa attcgcgtta aatttttgtt aaatcagctc      60

attttttaac caataggccg aaatcggcaa aatcccttat aaatcaaaag aatagaccga      120

gatagggttg agtgttgttc cagtttggaa caagagtcca ctattaaaga acgtggactc      180

caacgtcaaa gggcgaaaaa ccgtctatca gggcgatggc ccactacgtg aaccatcacc      240

ctaatcaagt tttttggggt cgaggtgccg taaagcacta aatcggaacc ctaaagggag      300

cccccgattt agagcttgac ggggaaagcc ggcgaacgtg gcgagaaagg aagggaagaa      360

agcgaaagga gcgggcgcta gggcgctggc aagtgtagcg gtcacgctgc gcgtaaccac      420

cacacccgcc gcgcttaatg cgccgctaca gggcgcgtcc cattcgccat tcaggctgcg      480

caactgttgg gaagggcgat cggtgcgggc ctcttcgcta ttacgccagc tggcgaaagg      540

gggatgtgct gcaaggcgat taagttgggt aacgccaggg ttttcccagt cacgacgttg      600

taaaacgacg gccagtgagc gcgcgtaata cgactcacta tagggcgaat tggagctcca      660

ccgcggtggc ggccgctcta gaaataattt tgtttaactt tactgatgga cagaattaat      720

ggatggctat ctcaatcaag accccagaag atatcgaaaa aatgcgcgtc gctggccgac      780

tggctgccga agtgctggag atgatcgaac cgtatgttaa accgggcgtc agcaccggcg      840

agctggatcg catctgtaat gattacattg ttaatgaaca acacgcggtt tctgcctgcc      900

tcggctatca cggctatccg aaatccgttt gcatctctat taatgaagtg gtgtgccacg      960

gtatcccgga cgatgctaag ctgctgaaag atggcgatat cgttaacatt gatgtcaccg     1020

taatcaaaga tggtttccac ggcgatacct cgaaaatgtt tatcgtcggt aagccgacca     1080

tcatgggcga acgtctgtgc cgcatcacgc aagaaagcct gtacctggcg ctacgcatgg     1140

taaaaccagg cattaatctg cgcgaaatcg gtgcggcgat tcagaaattt gtcgaagcag     1200

aaggcttctc cgtcgttcgt gaatattgcg gacacggtat tggtcgcggc ttccatgaag     1260

aaccgcaggt gctgcactat gactcccgtg aaaccaacgt cgtactgaaa cctgggatga     1320

cgttcaccat cgagccaatg gtcaacgcgg gtaaaaaaga gatccgcacc atgaaagatg     1380

gctggacggt aaaaaccaaa gatcgcagct tgtctgcaca atatgagcat actattgtgg     1440

tgactgataa cggctgcgaa attctgacgc tacgcaagga tgacaccatc ccggcgataa     1500

tctcgcacga cgaataatta actttaagaa ggagatatac atatggctag catgactggt     1560

ggacagcaaa tgggtcgcgg atccggctgc taacaaagcc cgaaaggaag ctgagttggc     1620

tgctgccacc gctgagcaat aactagcata accccttggg gcctctaaac gggtcttgag 1680
gggttttttg ctgaaaggag gaactatatc cggaattcga tatcaagctt atcgataccg 1740
tcgacctcga gggggggccc ggtacccagc ttttgttccc tttagtgagg gttaattgcg 1800
cgcttggcgt aatcatggtc atagctgttt cctgtgtgaa attgttatcc gctcacaatt 1860
ccacacaaca tacgagccgg aagcataaag tgtaaagcct ggggtgccta atgagtgagc 1920
taactcacat taattgcgtt gcgctcactg cccgctttcc agtcgggaaa cctgtcgtgc 1980
cagctgcatt aatgaatcgg ccaacgcgcg gggagaggcg gtttgcgtat tgggcgctct 2040
tccgcttcct cgctcactga ctcgctgcgc tcggtcgttc ggctgcggcg agcggtatca 2100
gctcactcaa aggcggtaat acggttatcc acagaatcag gggataacgc aggaaagaac 2160
atgtgagcaa aaggccagca aaaggccagg aaccgtaaaa aggccgcgtt gctggcgttt 2220
ttccataggc tccgcccccc tgacgagcat cacaaaaatc gacgctcaag tcagaggtgg 2280
cgaaacccga caggactata aagataccag gcgtttcccc ctggaagctc cctcgtgcgc 2340
tctcctgttc cgaccctgcc gcttaccgga tacctgtccg cctttctccc ttcgggaagc 2400
gtggcgcttt ctcatagctc acgctgtagg tatctcagtt cggtgtaggt cgttcgctcc 2460
aagctgggct gtgtgcacga accccccgtt cagcccgacc gctgcgcctt atccggtaac 2520
tatcgtcttg agtccaaccc ggtaagacac gacttatcgc cactggcagc agccactggt 2580
aacaggatta gcagagcgag gtatgtaggc ggtgctacag agttcttgaa gtggtggcct 2640
aactacggct acactagaag gacagtattt ggtatctgcg ctctgctgaa gccagttacc 2700
ttcggaaaaa gagttggtag ctcttgatcc ggcaaacaaa ccaccgctgg tagcggtggt 2760
ttttttgttt gcaagcagca gattacgcgc agaaaaaaag gatctcaaga agatcctttg 2820
atcttttcta cggggtctga cgctcagtgg aacgaaaact cacgttaagg gattttggtc 2880
atgagattat caaaaaggat cttcacctag atccttttaa attaaaaatg aagttttaaa 2940
tcaatctaaa gtatatatga gtaaacttgg tctgacagtt accaatgctt aatcagtgag 3000
gcacctatct cagcgatctg tctatttcgt tcatccatag ttgcctgact ccccgtcgtg 3060
tagataacta cgatacggga gggcttacca tctggcccca gtgctgcaat gataccgcga 3120
gacccacgct caccggctcc agatttatca gcaataaacc agccagccgg aagggccgag 3180
cgcagaagtg gtcctgcaac tttatccgcc tccatccagt ctattaattg ttgccgggaa 3240
gctagagtaa gtagttcgcc agttaatagt ttgcgcaacg ttgttgccat tgctacaggc 3300
atcgtggtgt cacgctcgtc gtttggtatg gcttcattca gctccggttc ccaacgatca 3360
aggcgagtta catgatcccc catgttgtgc aaaaaagcgg ttagctcctt cggtcctccg 3420
atcgttgtca gaagtaagtt ggccgcagtg ttatcactca tggttatggc agcactgcat 3480
aattctctta ctgtcatgcc atccgtaaga tgcttttctg tgactggtga gtactcaacc 3540

aagtcattct gagaatagtg tatgcggcga ccgagttgct cttgcccggc gtcaatacgg 3600

gataataccg cgccacatag cagaacttta aaagtgctca tcattggaaa acgttcttcg 3660

gggcgaaaac tctcaaggat cttaccgctg ttgagatcca gttcgatgta acccactcgt 3720

gcacccaact gatcttcagc atcttttact ttcaccagcg tttctgggtg agcaaaaaca 3780

ggaaggcaaa atgccgcaaa aaagggaata agggcgacac ggaaatgttg aatactcata 3840

ctcttccttt ttcaatatta ttgaagcatt tatcagggtt attgtctcat gagcggatac 3900

atatttgaat gtatttagaa aaataaacaa ataggggttc cgcgcacatt tccccgaaaa 3960

gtgccac 3967

<210> 3
<211> 432
<212> DNA
<213> Homo sapiens

<400> 3
atggttctgt ctccggctga caaaactaac gttaaagctg cttggggtaa agttggtgct 60

catgctggtg aatacggtgc tgaagctctg gaacgtatgt tcctgtcttt cccgactact 120

aaaacttact tcccgcattt cgacctgtct catggttctg ctcaggttaa aggtcatggt 180

aaaaaagttg ctgacgctct gactaacgct gttgctcatg ttgacgacat gccgaacgct 240

ctgtctgctc tgtctgacct gcatgctcat aaactgcgtg ttgacccggt taacttcaaa 300

ctgctgtctc attgcctgct ggttactctg gctgctcatc tgccggctga attcactccg 360

gctgttcatg cttctctgga caaattcctg gcttctgttt ctactgttct gacttctaaa 420

taccgttaat ga 432

<210> 4
<211> 447
<212> DNA
<213> Homo sapiens

<400> 4
atggttcatc tgactccgga agaaaaatct gctgttactg ctctgtgggg taaagttaac 60

gttgacgaag ttggtggtga agctctgggt cgtctgctgg ttgtttaccc gtggactcag 120

cgtttcttcg aatctttcgg tgacctgtct actccggacg ctgttatggg taacccgaaa 180

gttaaagctc atggtaaaaa agttctgggt gctttctctg acggtctggc tcatctggac 240

aacctgaaag gtactttcgc tactctgtct gaactgcatt gcgacaaact gcatgttgac 300

ccggaaaact tccgtctgct gggtaacgtt ctggtttgcg ttctggctca tcatttcggt 360

aaagaattca ctccgccggt tcaggctgct taccagaaag ttgttgctgg tgttgctaac 420

gctctggctc ataaatacca ttaatga 447

## Description

### Technical Field

The present invention relates to a vector including a methionine aminopeptidase gene to be used in synthesis of a protein by gene recombination technology. More specifically, the present invention relates to a high-copy number, high-expression vector for producing an unmodified protein where an N-terminal methionine residue (hereinafter, referred to as first methionine, in some cases) in a synthesized protein is removed in a synthesis reaction system; a method of producing the same; a transformant having the vector; and a method of producing a desired protein using the transformant.

### Background Art

A conventional method of producing a protein in a large quantity by gene recombination technology includes proliferating *Escherichia coli* integrated with a gene encoding the protein in a vector to amplify a copy of the coding gene; integrating the amplified gene into a vector capable of expressing a protein; and introducing the vector into a host such as *Escherichia coli* to express the protein, and the method requires a large amount of labor.

Therefore, a high-copy number, high-expression vector that can be used for producing a target protein in a simple manner has been developed as a high-copy number, high-expression vector that can be used for producing a protein in a large quantity and in a simple manner, which is obtained by integrating a sequence element for copying of a gene and a sequence element for expression of a protein into one vector (see Patent Document 1).

On the other hand, in protein synthesis processes in a living body, first, a protein having a first methionine corresponding to an initiation codon is synthesized, but in many cases, the protein having a first methionine does not have an original activity of a natural protein where the first methionine has been removed. That is, there are many proteins that become proteins having activity only by removing the first methionine with methionine aminopeptidase (hereinafter, abbreviated as MAP, in some cases) after transcription/translation of DNAs and expression of the proteins.

Therefore, there has been developed a technology to integrate a MAP gene into a vector to be used for obtaining a transformant for expression of hemoglobin, for example, and simultaneously remove methionine residues with MAP in an expression system (see Non-Patent Document 1).

However, when producing a protein using a transformant obtained by a high-copy-number, high-expression vector as described in Patent Document 1, a technology to simultaneously remove methionine residues in a produced protein in the expression system is not known.

In order to express proteins in a large quantity from various genes derived from prokaryotes and eukaryotes, a high-copy-number, high-expression vector has been developed. However, in the case where a protein to be targeted (protein to be produced, hereinafter, referred to as target protein) exhibits its functions by removing a first methionine, a protein that is expressed with a high-copy-number, high-expression vector and has a first methionine has different functions and activity from those of a natural protein.
Patent Document 1: JP 2004-208647 A

Non-Patent Document 1: Tong-Jian Shen et al., Proc. Natl. Acad. Sci. USA, Vol. 90, pp.8108-8112, September 1993, Biochemistry

### Disclosure of the Invention

### Problem to be solved by the Invention

An object of the present invention is to provide a high-copy number, high-expression vector capable of producing a protein having a satisfactory function/activity of the same level as that of a natural form of the protein, in a large quantity and in a simple manner.

### Means for solving the Problem

The inventors of the present invention have made extensive studies to solve the above-mentioned problems and have discovered that the above-mentioned problems can be solved by integrating a MAP gene into a high-copy number, high-expression vector, thereby completing the present invention.

The present invention relates to:
(1) a vector including (A) a target gene or a cloning site where the target gene is inserted, (B) a sequence element necessary for high copying of the target gene, (C) a sequence element necessary for expression of the target gene, and (D) a methionine aminopeptidase gene;
(2) a vector including (A) a target gene, (B) a sequence element necessary for high copying of the target gene, (C) a sequence element necessary for expression of the target gene, and (D) a methionine aminopeptidase gene;
(3) a vector including (A) a cloning site where a target gene is inserted, (B) a sequence element necessary for high copying of the target gene, (C) a sequence element necessary for the expression of the target gene, and (D) a methionine aminopeptidase gene;
(4) the vector according to Item 1, in which: (C) the sequence element necessary for the expression of the target gene includes (C-1) a promoter, (C-2) a ribosome binding site, and (C-3) a transcription termination site; and (A) the target gene or cloning site of the target gene is present between (C-2) the ribosome binding site and (C-3) the transcription termination site;
(5) the vector according to Item 1, including a base sequence having a promoter, a ribosome binding site, the methionine aminopeptidase gene, a ribosome binding site, the target gene or cloning site of the target gene, and a transcription termination site in the stated order from the transcription upstream side;
(6) the vector according to Item 4, in which (C-2) the ribosome binding site and (C-3) the transcription termination site include a sequence derived from pET3a;
(7) the vector according to Item 1, in which (B) the sequence element necessary for high copying of the target gene includes a base sequence including a replication origin suitable for a host;
(8) the vector according to Item 1, in which (B) the sequence element necessary for high copying of the target gene includes a base sequence having pUCori or fl(+)ori;
(9) the vector according to Item 1, in which (B) the sequence element necessary for high copying of the target gene includes a base sequence derived from pBluescriptII KS(+);
(10) the vector according to Item 1, in which (B) the sequence element necessary for high copying of the target gene is included in a fragment obtained by cleaving DNA derived from pBluescriptII KS(+) with EcoRI and XbaI;
(11) the vector according to Item 1, including (C-1) a T7 promoter sequence, (C-2) an SD sequence, (D) the methionine aminopeptidase gene, (C-2) an SD sequence, (A) an NdeI/BamHI cloning site where a target gene is inserted, and (C-3) a transcription termination site in the stated order from the transcription upstream side;
(12) the vector according to Item 1, in which (A) the target gene is a gene encoding a protein present in blood or a protein involved in oxygen transport;
(13) the vector according to Item 1, in which (A) the target gene is a gene encoding hemoglobin, albumin, or a modified compound thereof;
(14) the vector according to Item 1, in which (A) the target gene is a gene encoding hemoglobin;
(15) a method of producing a vector, including the steps of: inserting into EcoRI and XbaI sites of pBluescriptII KS(+) a DNA fragment obtained by digesting pET3a including an SD sequence, an NdeI/BamHI cloning site where a target gene is inserted, and a transcription termination site with XbaI and EcoRI; and inserting into XbaI and NdeI sites of the resultant vector a DNA fragment having an SD sequence, a methionine aminopeptidase gene, and an SD sequence in the stated order from the transcription upstream side;
(16) a transformant introduced with the vector according to Item 2; and
(17) a method of producing a protein using the transformant according to Item 16.

### Effect of the Invention

Cultivation of a transformant having a vector of the present invention can produce the same protein as a natural form of the protein, where a first methionine has been removed, in a large quantity and in a simple manner. For example, it is possible to produce human adult hemoglobin, which is a major ingredient of an artificial oxygen transporter, in a state where a first methionine has been removed as the natural form.

### Brief Description of the Drawings

[Fig. 1] A figure showing an amino acid sequence and a base sequence of human adult hemoglobin α-chain.
[Fig. 2] A figure showing an amino acid sequence and a base sequence of human adult hemoglobin β-chain.
[Fig. 3] A figure showing a base sequence in the vicinity of an NdeI/BamHI cloning site of pET3a.
[Fig. 4] A figure showing a base sequence in the vicinity of a multicloning site of pBluescriptII KS(+).
[Fig. 5] A figure illustrating a method of producing a high-copy-number, high-expression vector (pBEX) to be used in production of a vector (pMAX) of the present invention in the Examples.
[Fig. 6] A figure illustrating a method of producing a vector (pMAX) of the present invention by integrating a DNA fragment having an XbaI recognition sequence, an SD sequence, a MAP gene, an SD sequence, and an NdeI recognition sequence in the stated order from the transcription upstream side into a XbaI/NdeI cloning site of pBEX.

### Best Mode for carrying out the Invention

A vector of the present invention includes a sequence element necessary for the high copying of a target gene, a sequence element necessary for the expression of the target gene, and a MAP gene. The vector of the present invention can simultaneously perform not only copying and expression of a target gene but also removal of N-terminal methionine of an expressed protein.

In the present invention, the target gene is a gene that expresses a target protein. The type of the gene is not particularly limited, but the gene is generally a gene encoding a target protein. Meanwhile, a gene encoding a target protein may be a gene modified so as to be optimal for expression in a host. In addition, the gene may be a gene derived from a living body or an artificially synthesized gene.

Examples of the target protein include proteins present in a living body, and specific examples thereof include hemoglobin, albumin, and modified compounds thereof. The protein is preferably a protein present in blood or a protein involved in oxygen transport, more preferably hemoglobin, most preferably human adult hemoglobin.

The protein in the present invention includes not only complete natural proteins but also mutant proteins and partial polypeptides, and it is preferably a natural protein having complete activity.

The base sequence of the target gene is preferably a sequence containing no intron (intervening sequence).

Specific examples of the target gene include a gene having base sequences described in SEQ ID NOS: 3 and 4.

The sequence element necessary for the high copying of the target gene is a base sequence necessary for replication of the target gene, and examples thereof include a base sequence including a replication origin suitable for a host. In the case where the host is *Escherichia coli,* examples of the replication origin suitable for a host include pUCori and fl(+)ori.

The sequence element necessary for replication is preferably a base sequence derived from pBluescriptII KS(+) (manufactured by Stratagene), and is more preferably included in a DNA fragment obtained by cleaving pBluescriptII KS (+) with EcoRI and XbaI.

The sequence element necessary for the expression of the target gene is a sequence including a promoter, a ribosome binding site, and a transcription termination site, and in general, the sequence element includes a target gene or a cloning site between the ribosome binding site and the transcription termination site.

More preferably, the ribosome binding site and transcription termination site have sequences derived from pET3a.

The promoter is not particularly limited as long as it can act in a host to be used for the expression of a vector of the present invention. Examples of a promoter that can act in bacterial cells include a T7, lac, trp, or tac promoter of *Escherichia coli* and a PR or PL promoter of phage lambda or the like. Examples of a promoter that can act in yeast host cells include a promoter derived from a yeast glycolytic gene, an alcohol dehydrogenase gene promoter, a TPI1 promoter, and an ADH2-4c promoter or the like. Examples of a promoter that can act in filamentous fungus cells include an ADH3 promoter or a tpiA promoter or the like. Examples of a promoter that can act in mammal cells include an SV40 promoter, a metallothionein gene promoter, or an adenovirus type 2 major late promoter or the like. Examples of a promoter that can act in insect cells include a polyhedrin promoter, a P10 promoter, an Autographa californica polyhedrosis basic protein promoter, a baculovirus immediate-early gene 1 promoter, or a baculovirus 39K delayed-early gene promoter.

Of those, the T7 promoter and tac promoter are preferred, and the T7 promoter is particularly preferred.

Examples of the ribosome binding site include an SD sequence.

In the present invention, a sequence derived from a plasmid pET3a or the like may be used as the ribosome binding site.

Examples of the transcription termination site include a polyadenylation site.

In the present invention, a sequence derived from a plasmid pET3a or the like may be used as the transcription termination site.

The vector of the present invention is a vector that is produced by gene recombination technology and can transform a host cell.

In general, the vector of the present invention has a promoter, a ribosome binding site, a MAP gene, a ribosome binding site, and a target gene or a cloning site of a target gene, and a transcription termination site in the stated order from the transcription upstream side.

Examples of a vector of the present invention include: vectors derived from chromosomes, episomes, bacterial plasmids derived from viruses, yeast plasmids, papovaviruses, vacciniaviruses, adenoviruses, adeno-associated viruses, fowlpox viruses, pseudorabies viruses, and retroviruses; vectors derived from bacteriophage; vectors derived from transposon; and vectors derived from a combination of the above-mentioned origins . Of those, a vector derived from a bacterial plasmid is preferred.

In production of a vector of the present invention, base sequences derived from two or more bacterial plasmids or the like may be used in combination. '

A vector of the present invention may have a cloning site. The cloning site is preferably located between a ribosome binding site and a transcription termination site. Examples of the cloning site include an NdeI/BamHI cloning site.

In the case where a vector has a cloning site, a recombinant expression vector for expression of a target gene may be produced by inserting the target gene into a cloning site.

A vector of the present invention may further include an introduction marker. Examples of the introduction marker include a drug resistance gene. Examples of the drug resistance gene include antibiotic resistance genes such as ampicillin resistance gene, neomycin resistance gene, and tetracycline resistance gene.

Examples of the vector of the present invention include a vector including a T7 promoter, an SD sequence, a MAP gene, an NdeI/BamHI cloning site, and a transcription termination site.

More specifically, examples of the vector include a vector having the base sequence of SEQ ID NO: 2, produced by inserting a base sequence that is derived from pET3a including an NdeI/BamHI cloning site and a transcription termination site and a base sequence including an SD sequence and a MAP gene into EcoRI and XbaI sites in pBluescriptII KS(+).

A method of integrating a DNA fragment including the sequence element necessary for the high copying of a target gene, the sequence element necessary for the expression of the target gene, and the MAP gene into a vector may be any of various methods to be used in gene recombination technology, and examples thereof include a method of adding a ligase to a mix solution of the vector and DNA fragments obtained by a treatment with various restriction enzymes to bind the vector to the DNA fragments.

Introduction of a vector of the present invention into various host cells can produce a transformant for expressing a target gene.

Examples of the host cells include prokaryotic cells of bacteria such as *Escherichia coli, Streptomyces, Bacillus subtilis, Streptococcus,* and *Staphylococcus;* cells of fungi such as yeast and *Aspergillus;* and insect cells such as *Drosophila* S2 and *Spodoptera* Sf9. In the present invention, use of *Escherichia coli* is particularly preferred because of its high proliferating ability.

Introduction of a vector into host cells can be performed by a conventional method. Examples of the method include various methods such as the competent cell method, protoplast method, calcium phosphate coprecipitation method, electroporation method, microinjection method, liposome fusion method, particle gun method, DEAE-dextran-mediated transfection, transvection, cationic lipid-mediated transfection, transduction, scrape loading, ballistic introduction, and infection, and anymethodmay be employed depending on a host to be used.

Meanwhile, the present invention provides a method of producing a protein using the transformant.

That is, if the transformant (preferably, *Escherichia coli*) obtained by introduction of a vector of the present invention is cultivated, a target protein can be produced in a large quantity in a culture product.

A medium for culture of a transformant is known, and examples thereof include a nutrient medium such as YPD medium; a minimal medium such as MB medium; BMMY medium; and BMGY medium.

The transformant is cultivated generally at about 16 to 42 °C, preferably at about 25 to 37 °C for about 8 to 168 hours, preferably for about 24 to 120 hours. The transformant may be subjected to shaking culture or static culture, and if necessary, the culture may be performed with stirring or aeration. In the case of using *Escherichia coli* as a transformant, *Escherichia coli* may be cultivated preferably at 30 to 40°C in a known medium such as TB medium.

Meanwhile, in order to increase the amount of production of a target protein, culture is preferably performed in a medium supplemented with a synthetic substrate of the target protein. In the case where the target protein is hemoglobin, culture is preferably performed in a medium supplemented with aminolevulinic acid as a synthetic substrate.

A target protein can be obtained by isolating a protein from the culture medium by a known method, and if necessary, purifying the product.

Examples of a method of isolating/purifying a fused protein produced in a culture include a knownmethod using a difference in solubility, such as a salting-out or solvent precipitation method; dialysis; a method using a difference in molecular weight, such as ultrafiltration, or gel electrophoresis; a method using a difference in charge, such as ion-exchange chromatography; a method using specific affinity, such as affinity chromatography; a method using a difference in hydrophobic property, such as reversed-phase high performance liquid chromatography; and a method using a difference in isoelectric point, such as isoelectric focusing.

More specific examples of the isolation method include a method including collecting bacterial cells from a culture medium by centrifugation; suspending the cells in phosphate buffer or the like; homogenizing the cells by sonication; centrifuging the resultant cells; collecting the supernatant; dialyzing the supernatant against phosphate buffer or the like; purifying the product using a cation-exchange column; passing the product through an anion-exchange column at pH 7.4; and purifying the product using an anion-exchange column at pH 8.5.

The resultant target protein is preferably concentrated and stored in a mix solution of a buffer/glycerin, for example.

The above-mentioned step of isolating/purifying a target protein must be performed under a temperature condition that causes no denaturation of the target protein, and an optimum temperature condition must be set because MAP has high activity at a temperature higher than room temperature.

Examples of a method of identifying an isolated/purified fused protein include the known Western blotting method and activity measurement method. Meanwhile, the structure of a purified fused protein can be determined by an amino acid analysis, an amino-terminal analysis, a primary structural analysis, etc.

A protein obtained using a vector of the present invention can be used for various known protein applications without modification or after chemical modification, if necessary, and the protein can be administered to a living body for medical purposes, for example.

In the case where the protein is one that acts as an oxygen transporter such as hemoglobin, it can be administered as an erythrocyte substitute. In the case where the protein is administered as an erythrocyte substitute, the protein may be encapsulated in a liposome or dispersed in an emulsion before administration.

Meanwhile, a protein produced using a vector of the present invention can be administered as a pharmaceutical composition containing an electrolyte and/or a plasma volume expander and/or a pH adjuster and/or an antioxidant.

### Example 1

### (1) Design of DNA encoding oxygen-transporting protein (target gene)

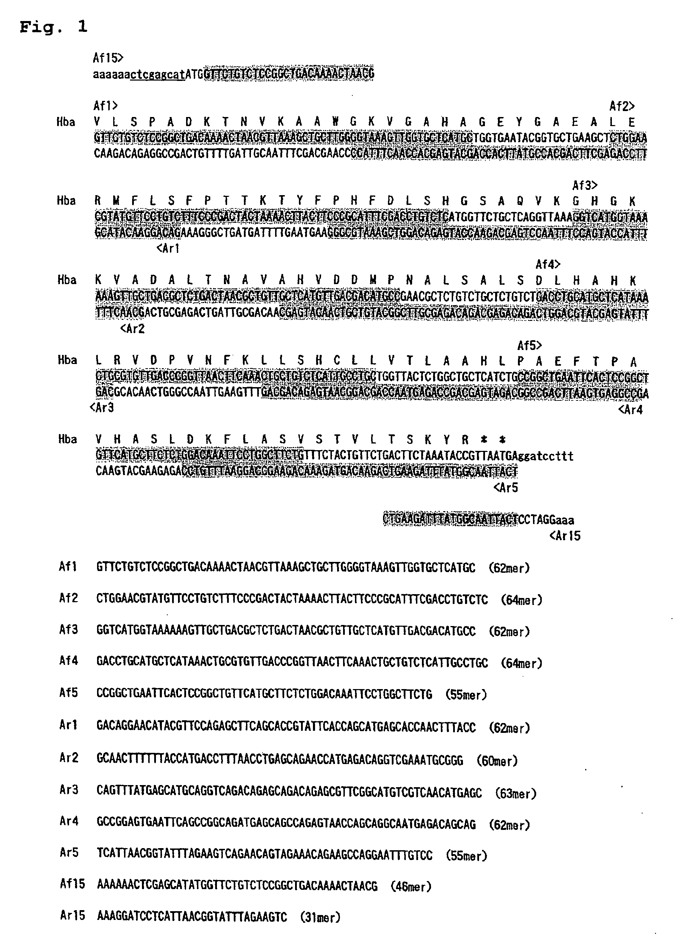

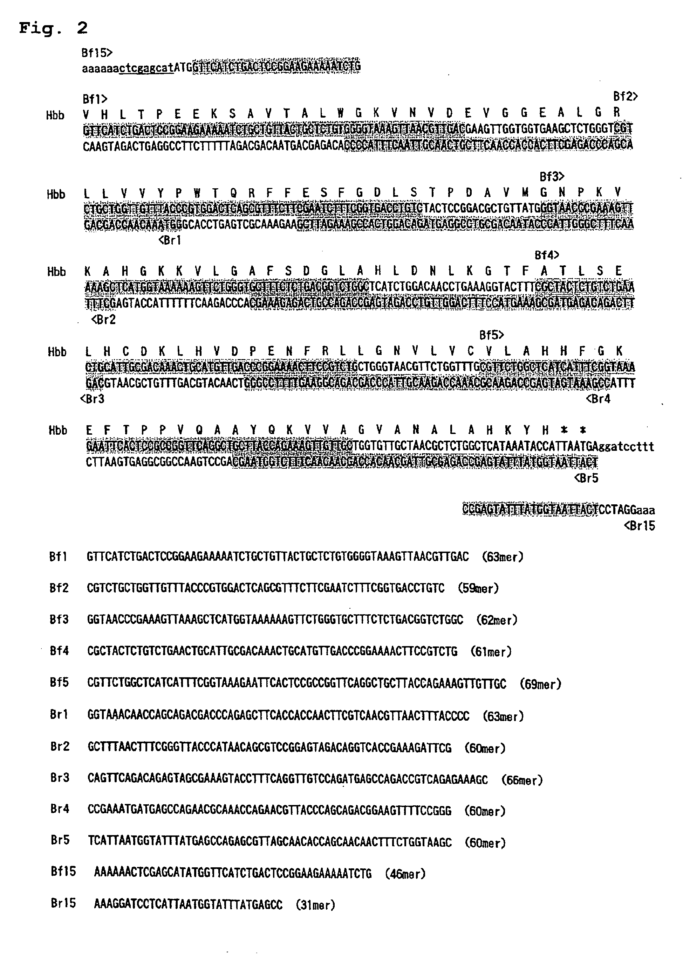

A target gene for an oxygen-transporting protein, i.e., human adult hemoglobin, was designed. The base sequence of the target gene was designed using a codon allowing high expression in *Escherichia coli* without modifying the amino acid sequence of human adult hemoglobin. The amino acid sequence and base sequence of the designed target gene (SEQ ID NOS: 3 and 4) are shown in Fig. 1 (α-chain) and Fig. 2 (β-chain) .

### (2) PCR

Based on the design of (1) above, a DNA oligomer was synthesized. A method of synthesizing fragments of a gene encoding α-chain of human adult hemoglobin will be described. First, oligomers shown in Fig. 1: Af1, Af2, Af3, Af4, Af5, Bf1, Bf2, Bf3, Bf4, and Bf5 were mixed in equal molar amounts to perform primary PCR. A small amount of the solution was separated, and Afl5 and Ar15 were added to the solution to perform secondary PCR.

### (3) Proliferation and cloning using Escherichia coli

PCR-amplified fragments were cleaved with restriction enzyme sites (NdeI and XheI) introduced to the terminal, and ligated to a plasmid pBluescriptII SK(+) (manufactured by Stratagene) treated with the same enzymes. A host of *Escherichia coli* XL-1 Blue MRF' (manufactured by Stratagene) is transformed with the plasmid, and the transformant was inoculated into an LB plate containing ampicillin. Culture was performed at 37°C overnight, and colonies of *Escherichia coli* were separated, followed by liquid culture using LB medium containing ampicillin. The bacterial cells were collected, and plasmids were extracted (using Miniprep manufactured by Promega), followed by determination of the base sequences using a DNA sequencer (manufactured by LI-COR Biosciences). It was confirmed that desired fragments without unexpected mutation were obtained.

In the same way as described above, a gene encoding β-chain of human adult hemoglobin was synthesized (Fig. 2).

### (4) High-copy number, high-expression vector

A cloning vector, pBluescriptII KS(+) (manufactured by Stratagene), and an expression vector, pET3a (manufactured by Stratagene), were used to produce a high-copy number, high-expression vector.

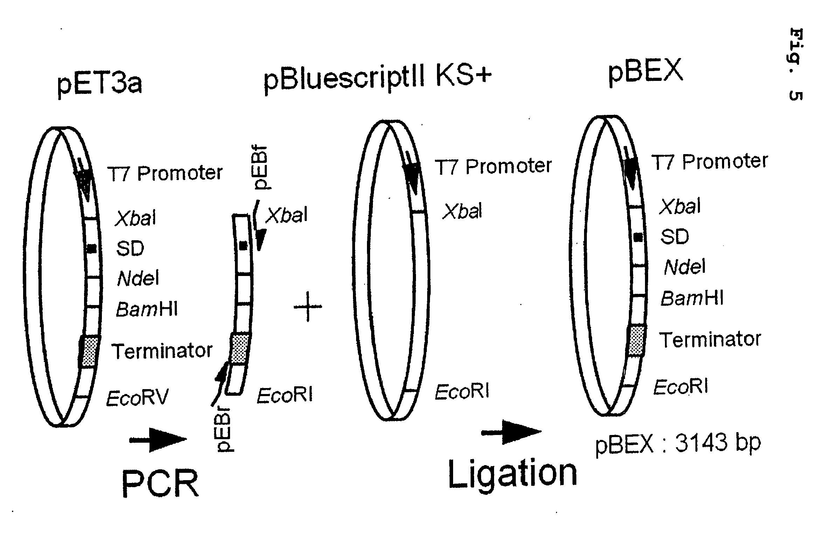

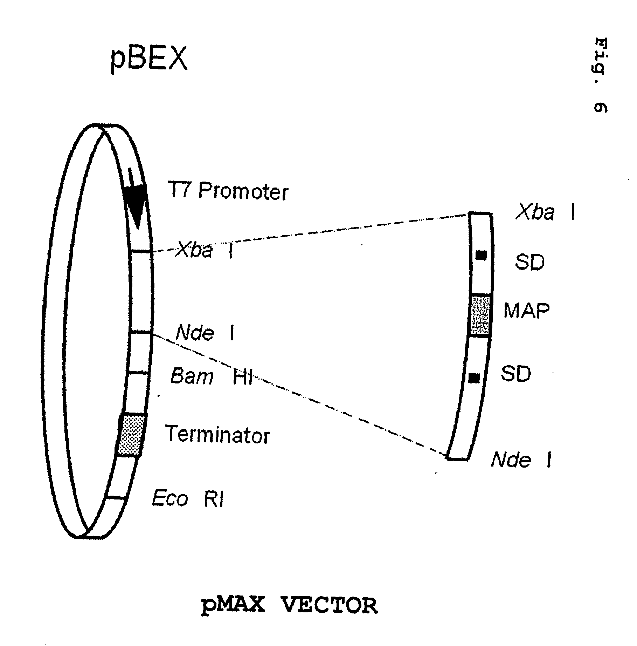

The outlines of a method of constructing pMAX are shown in Figs. 5 and 6.

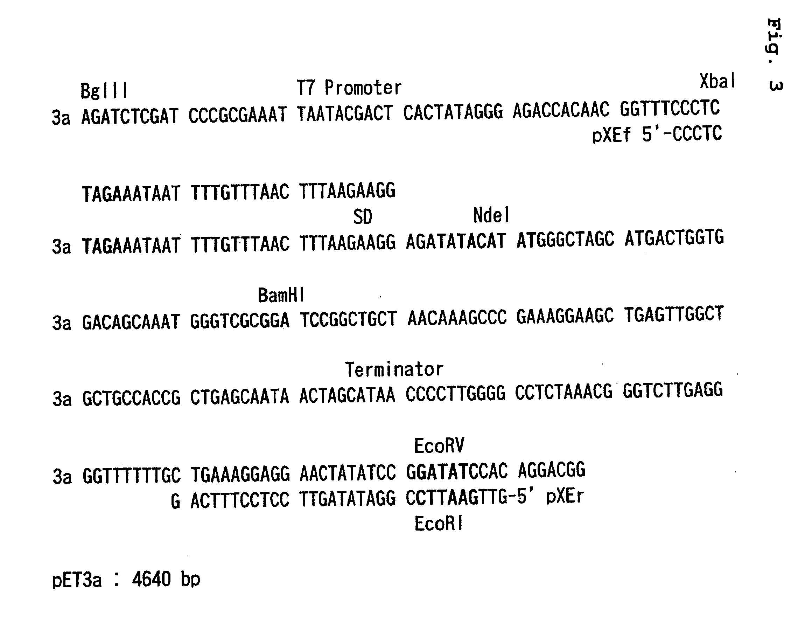

First, primers pXEf and pXEr (Fig. 3) which include XbaI and EcoRV sites of pET3a, respectively, were designed, and PCR was performed using them to amplify target sequences. In this case, an EcoRI site was integrated into pXEr instead of EcoRV. This is because EcoRV provides a blunt end while EcoRI provides a sticky end and therefore facilitates next ligation. The PCR yielded a fragment including an SD sequence, an NdeI/BamHI cloning site, and a terminator of pET3a. The fragment was treated with XbaI and EcoRI and ligated to pBluescriptII KS (+) treated with the same restriction enzymes, to thereby yield a vector (pBEX).

Next, a DNA fragment obtained by adding an SD sequence, XbaI, and NdeI sequences to the upstream and downstream of a MAP gene of *Escherichia coli* JM 109 was amplified by PCR using the DNA fragment as a template. The amplified products were treated with XbaI and NdeI and ligated to a vector pBEX treated with the same restriction enzymes, to thereby yield a vector (pMAX) of the present invention. Hereinafter, a specific method of constructing a vector pMAX will be described.

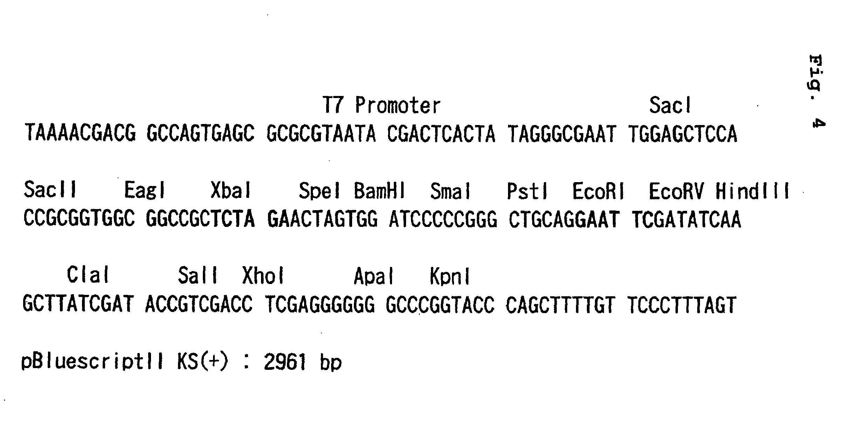

Fig. 3 shows a base sequence in the vicinity of an NdeI/BamHI cloning site of pET3a. DNA including a ribosome binding site (SD sequence) necessary for translation and a terminator site necessary for termination of the translation (from the XbaI site to the EcoRI site of Fig. 3) was amplified using primers (XEf and XEr of Fig. 3). In this case, the primer XEr includes an EcoRI recognition sequence instead of a part corresponding to an EcoRV recognition sequence of pET3a so as to be ligated to pBluescriptII KS (+). This fragment was digested with XbaI and EcoRI and inserted into a multicloning site of pBluescriptII KS (+). The base sequence in the vicinity of the multicloning site of pBluescriptII KS(+) is shown in Fig. 4.

Fragments amplified by pET3a were integrated into pBluescriptII KS(+) to ligate a T7 promoter derived from pBluescriptII KS(+), and an SD sequence, an NdeI recognition sequence, a BamHI recognition sequence, and a transcription termination site derived from pET3a. A vector having a base sequence with a full length of 3,143 base pairs described in SEQ ID NO: 1 (hereinafter, referred to as pBEX), in addition to a base sequence of pBluescriptII KS(+), was produced.

pBluescriptII KS(+) is frequently used as a high-copy-number vector and has a multicloning site integrated with various restriction enzyme cleavage site (Fig. 4). On the other hand, a pET-based vector has a T7 promoter, an SD sequence, and a terminator necessary for induction/expression of a protein, and pET3a clones a target gene in an NdeI/BamHI cloning site downstream of an SD sequence to achieve the high expression of a protein (Fig. 3). However, the copy number of the vector per cell is small. pBEX is a high-copy-number, high-expression vector that takes advantages of the both vectors.

Next, a MAP gene was isolated from chromosome of *Escherichia coli* JM109 by PCR to produce a DNA fragment having a XbaI recognition sequence, an SD sequence, a MAP gene, an SD sequence, and an NdeI recognition sequence in the stated order from the transcription upstream side (see Fig. 6), and PCR amplification was performed using the DNA fragment as a template. PCR products were treated with restriction enzymes (XbaI and NdeI) and integrated into a XbaI/NdeI cloning site of pBEX, to thereby yield a novel vector having a base sequence with a full length of 3, 967 base pairs described in SEQ ID NO: 2 (hereinafter, referred to as pMAX).

The resultant pMAX was introduced into XI,-1 Blue MRF' , and bacterial cells containing pMAX were selected and used as a stock.

For abundant expression using the vector pMAX, it is desirable to use *Escherichia coli* BL21 (DE3) that expresses T7 RNA polymerase as a host.

### (6) Mass production of recombinant hemoglobin (rHb) using pMAX vector

Total synthesis of a human hemoglobin gene (human Hb) gene modified to be expressed in *Escherichia coli* in a simple manner was performed in vitro. The gene modified to be expressed in *Escherichia coli* in a simple manner means a gene obtained by substituting a codon of a human Hb gene for a codon that is frequently expressed in *Escherichia coli* among a plurality of triplet codons corresponding to one amino acid. The substitution is known to increase the expression level of a target protein.

Next, a vector obtained by integrating a synthesized Hb gene into an NdeI/BamHI cloning site of pMAX was introduced into *Escherichia coli* BL21 Gold (DE3) by the calcium phosphate method. The resultant transformant was cultivated using LB medium at 37°C overnight and then inoculated into a fresh LB medium. 0.5 mM aminolevulinic acid was added thereto to perform mass culture at 37°C for 72 hours. Note that aminolevulinic acid is a synthetic substrate of heme, and addition of aminolevulinic acid can increase the expression level of rHb.

### (7) Purification of rHb

The bacterial cells were collected by centrifugation, and suspended in 20 mM phosphate buffer (pH 7.0), and homogenized by sonication.

The homogenized bacterial cells were centrifuged at 15,000 rpm for 60 minutes, and the supernatant was collected. The resultant supernatant was dialyzed against 20 mM phosphate buffer (pH 6) and purified using a SP Sepharose column (manufactured by Amersham Biosciences K.K.), and the eluent was passed through a Q-Sepharose column (manufactured by Amersham Biosciences K.K.) at pH 7.4. Further, the eluent was purified using a Q-Sepharose column at pH 8.5. The eluent was concentrated to exchange the buffer for 20 mM phosphate buffer (pH 7.4), and stored in CO atmosphere at 4°C.

### (8) Determination of protein

The final sample was subjected to SDS polyacrylamide gel electrophoresis, and a single band having an expected molecular weight of about 15, 000 was detected. Meanwhile, an N-terminal amino acid analysis revealed that the band corresponded to rHb as expected and that a first methionine was not detected. In addition, a mass spectrum confirmedmolecular weights of α- and β-chains to be 15, 126.0 and 15, 867. 5, respectively, which were the same as calculated values.

### Industrial Applicability

A vector of the present invention can be used for transforming various host cells so as to produce a target protein on a massive scale.

A protein obtained by a production method of the present invention can be used in various protein applications and also can be administered to a living body, in particular, for medical purposes because there is no probability of contamination due to a virus or the like.

## Claims

1. A vector comprising (A) a target gene or a cloning site where the target gene is inserted, (B) a sequence element necessary for high copying of the target gene, (C) a sequence element necessary for expression of the target gene, and (D) a methionine aminopeptidase gene.

2. A vector comprising (A) a target gene, (B) a sequence element necessary for high copying of the target gene, (C) a sequence element necessary for expression of the target gene, and (D) a methionine aminopeptidase gene.

3. A vector comprising (A) a cloning site where a target gene is inserted, (B) a sequence element necessary for high copying of the target gene, (C) a sequence element necessary for the expression of the target gene, and (D) a methionine aminopeptidase gene.

4. The vector (3) according to Claim 1, wherein
(C) the sequence element necessary for the expression of the target gene includes (C-1) a promoter, (C-2) a ribosome binding site, and (C-3) a transcription termination site; and
(A) the target gene or cloning site of the target gene is present between (C-2) the ribosome binding site and (C-3) the transcription termination site.

5. The vector according to Claim 1, comprising a base sequence having (C-1) a promoter, (C-2) a ribosome binding site, (D) the methionine aminopeptidase gene, (C-2) a ribosome binding site, (A) the target gene or cloning site of the target gene, and (C-3) a transcription termination site in the stated order from the transcription upstream side.

6. The vector (3) according to Claim 4, wherein (C-2) the ribosome binding site and (C-3) the transcription termination site include a sequence derived from pET3a.

7. The vector according to Claim 1, wherein (B) the sequence element necessary for high copying of the target gene includes a base sequence including a replication origin suitable for a host.

8. The vector according to Claim 1, wherein (B) the sequence element necessary for high copying of the target gene includes a base sequence having pUCori or fl(+)ori.

9. The vector according to Claim 1, wherein (B) the sequence element necessary for high copying of the target gene includes a base sequence derived from pBluescriptII KS(+).

10. The vector according to Claim 1, wherein (B) the sequence element necessary for high copying of the target gene is included in a fragment obtained by cleaving DNA derived from pBluescriptII KS(+) with EcoRI and XbaI.

11. The vector according to Claim 1, comprising (C-1) a T7 promoter sequence, (C-2) an SD sequence, (D) the methionine aminopeptidase gene, (C-2) an SD sequence, (A) an NdeI/BamHI cloning site where a target gene is inserted, and (C-3) a transcription termination site in the stated order from the transcription upstream side.

12. The vector according to Claim 1, wherein (A) the target gene is a gene encoding a protein present in blood or a protein involved in oxygen transport.

13. The vector according to Claim 1, wherein (A) the target gene is a gene encoding hemoglobin, albumin, or a modified compound thereof.

14. The vector according to Claim 1, wherein (A) the target gene is a gene encoding hemoglobin.

15. A method of producing a vector, comprising the steps of
inserting a DNA fragment obtained by digesting pET3a including an SD sequence, an NdeI/BamHI cloning site where a target gene is inserted, and a transcription termination site with XbaI and EcoRI into EcoRI and XbaI sites of pBluescriptII KS(+); and
inserting a DNA fragment having an SD sequence, a methionine aminopeptidase gene, and an SD sequence in the stated order from the transcription upstream side into XbaI and NdeI sites of the resultant vector.

16. A transformant introduced with the vector according to Claim 2.

17. A method of producing a protein, comprising using the transformant according to Claim 16.
